(19) Europäisches Patentamt
European Patent Office
Office européen des brevets

(11) Veröffentlichungsnummer : **0 169 474**
**B1**

(12) **EUROPÄISCHE PATENTSCHRIFT**

(45) Veröffentlichungstag der Patentschrift : 28.09.88

(21) Anmeldenummer : 85108863.3

(22) Anmeldetag : 16.07.85

(51) Int. Cl.⁴ : C 07 D249/08, C 07 D233/60, A 61 K 31/415, A 61 K 31/41, C 07 C131/00

(54) **Substituierte Hydroxyalkyl-azole, Verfahren zu ihrer Herstellung sowie diese enthaltende antimykotische Mittel.**

(30) Priorität : 27.07.84 DE 3427844

(43) Veröffentlichungstag der Anmeldung : 29.01.86 Patentblatt 86/05

(45) Bekanntmachung des Hinweises auf die Patenterteilung : 28.09.88 Patentblatt 88/39

(84) Benannte Vertragsstaaten : AT BE CH DE FR GB IT LI NL SE

(56) Entgegenhaltungen :
EP-A- 0 111 234
M.R.Siegel and H.D.Sirler "Antifungal Compounds", Marcel Dekker Inc. 1977, pp. 455-457
Die Akte enthält technische Angaben, die nach dem Eingang der Anmeldung eingereicht wurden und die nicht in dieser Patentschrift enthalten sind.

(73) Patentinhaber : BAYER AG
Konzernverwaltung RP Patentabteilung
D-5090 Leverkusen 1 Bayerwerk (DE)

(72) Erfinder : Elbe, Hans Ludwig, Dr.
Dasnöckel 59
D-5600 Wuppertal 11 (DE)
Erfinder : Stetter, Jörg, Dr.
Gellertweg 4
D-5600 Wuppertal 1 (DE)
Erfinder : Büchel, Karl Heinz, Prof. Dr.
Dabringhausener Strasse 42
D 5093 Burscheid (DE)
Erfinder : Schaller, Klaus, Dr.
Am Sonnenschein 38,
D 5600 Wuppertal 1 (DE)
Erfinder : Plempel, Manfred, Dr.
Zwengenbergerstrasse 3c
D 5657 Haan (DE)

EP 0 169 474 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte Hydroxyalkyl-azole, ein Verfahren zu ihrer Herstellung sowie diese enthaltende antimykotische Mittel.

Es ist bereits bekannt geworden, daß bestimmte Hydroxyalkyl-azolyl-Derivate gute antimykotische Eigenschaften aufweisen.

Bestimmte Hydroxyalkylazolyl-Derivate enthaltende fungizide Mittel sind aus der EP-A-111 234 bekannt.

M. R. Siegel et.al. beschreiben in « Antifungal Compounds », Marcel Dekker Inc. antimykotisch wirkende Verbindunge. Die Wirkung dieser Verbindungen ist jedoch nicht in allen Indikationen zufriedenstellend.

Gefunden wurden neue substituierte Hydroxyalkyl-azole der allgemeinen Formel

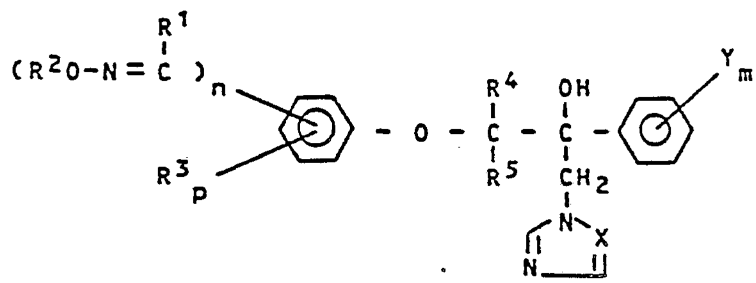

(I)

in welcher

$R^1$ für Wasserstoff, geradkettiges oder verzweigtes Alkyl mit 1 bis 10 Kohlenstoffatomen sowie für jeweils gegebenenfalls einfach bis dreifach, gleich oder verschieden substituiertes Phenyl oder Benzyl, wobei als Substituenten die Bedeutungen von Y infrage kommen ;

$R^2$ für Wasserstoff, geradkettiges oder verzweigtes Alkyl mit 1 bis 10 Kohlenstoffatomen, geradkettiges oder verzweigtes Alkenyl und Alkinyl mit jeweils 3 bis 10 Kohlenstoffatomen sowie für jeweils gegebenenfalls einfach bis dreifach, gleich oder verschieden substituiertes Phenyl und Benzyl, wobei als Substituenten die Bedeutungen von Y infrage kommen ;

n für die Zahlen 1 oder 2 ;

$R^3$ für die Bedeutungen von Y ;

p für die Zahlen 0, 1 oder 2 ;

$R^4$ für geradkettiges oder verzweigtes Alkyl mit 1 bis 4 Kohlenstoffatomen ;

$R^5$ für geradkettiges oder verzweigtes Alkyl mit 1 bis 4 Kohlenstoffatomen ;

X für ein Stickstoffatom oder die CH-Gruppe ;

Y für Halogen, Alkyl mit 1 bis 4 Kohlenstoffatomen, Cycloalkyl mit 5-7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen,

m für die Zahlen 0, 1, 2 oder 3 steht,

sowie deren Säureadditions-Salze.

Die Verbindungen der Formel (I) besitzen mindestens ein, gegebenenfalls zwei asymmetrische Kohlenstoffatome und können deshalb in verschiedenen optischen Isomerenformen anfallen.

Weiterhin wurde gefunden, daß man die substituierten Hydroxyalkyl-azole der Formel (I) erhält, wenn man Oxirane der Formel

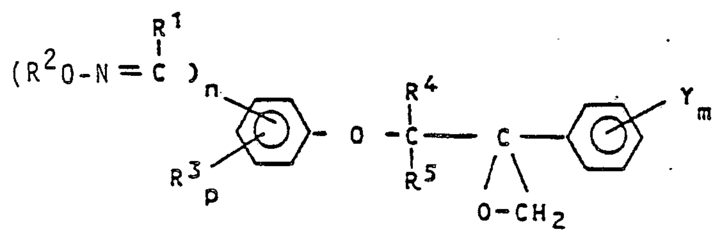

(II)

in welcher $R^1$, $R^2$, $R^3$, $R^4$, $R^5$, Y
und die Indizes m, n und p die oben angegebene Bedeutung haben,
mit Azolen der Formel

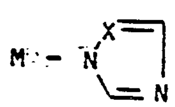

(III)

in welcher

X die oben angegebene Bedeutung hat und

M für Wasserstoff oder ein Alkalimetall steht,

in Gegenwart eines Verdünnungsmittels und gegebenenfalls in Gegenwart einer Base umsetzt.

An die so erhaltenen Verbindungen der Formel (I) kann gegebenenfalls anschließend eine Säure addiert werden.

Die neuen substituierten Hydroxyalkyl-azole der Formel (I) weisen starke antimykotische Eigenschaften auf. Dabei zeigen überraschenderweise die erfindungsgemäßen Verbindungen insbesondere eine bessere, therapeutisch nutzbare in-vivo-Wirksamkeit als die aus dem Stand der Technik bekannten chemisch ähnlichen Verbindungen. Die erfindungsgemäßen Stoffe stellen somit eine Bereicherung der Pharmazie dar.

Außerdem sind die neuen substituierten Hydroxyalkylazole interessante Zwischenprodukte. So können z. B. die Verbindungen der allgemeinen Formel (I) an der Hydroxygruppe in üblicher Weise in die entsprechenden Ether überführt werden. Weiterhin können durch Umsetzung mit z. B. Acylhalogeniden oder Carbamoylchloriden in prinzipiell bekannter Weise Acyl- oder Carbamoyl-Derivate der Verbindungen der allgemeinen Formel (I) erhalten werden.

Die erfindungsgemäßen substituierten Hydroxyalkylazole sind durch die Formel (I) allgemein definiert.

Besonders bevorzugt sind diejenigen Verbindungen der Formel (I), in denen

$R^1$ für Wasserstoff, geradkettiges oder verzweigtes Alkyl mit 1 bis 4 Kohlenstoffatomen sowie für jeweils gegebenenfalls einfach oder zweifach, gleich oder verschieden substituiertes Phenyl und Benzyl steht, wobei als Substituenten die Bedeutungen von Y infrage kommen ;

$R^2$ für Wasserstoff, geradkettiges oder verzweigtes Alkyl mit 1 bis 4 Kohlenstoffatomen, geradkettiges oder verzweigtes Alkenyl und Alkinyl mit jeweils 3 bis 4 Kohlenstoffatomen sowie für jeweils gegebenenfalls einfach oder zweifach, gleich oder verschieden substituiertes Phenyl und Benzyl steht, wobei als Substituenten die Bedeutungen von Y infrage kommen ;

n für die Zahlen 1 oder 2 steht ;

$R^3$ für die Bedeutungen von Y steht ;

p für die Zahlen 0, 1 oder 2 steht ;

$R^4$ für Methyl oder Ethyl steht ;

$R^5$ für Methyl oder Ethyl steht ;

X für ein Stickstoffatom oder die CH-Gruppe steht ;

Y für Fluor, Chlor, Brom, Methyl, Isopropyl, tert.-Butyl, Cyclohexyl, Methoxy, Methylthio, Trifluormethyl, Trifluormethoxy, Trifluormethylthio und

m für die Zahlen 0, 1 oder 2 steht.

Bevorzugte erfindungsgemäße Verbindungen sind auch Additionsprodukte aus Säuren und denjenigen substituierten Hydroxyalkyl-azolen der Formel (I), in denen die Substituenten $R^1$, $R^2$, $R^3$, $R^4$, $R^5$, X und Y sowie die Indizes m, n und p die Bedeutungen haben, die bereits vorzugsweise für die Substituenten sowie Indizies genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Im einzelnen seien außer den bei den Herstellungsbeispielen genannten Verbindungen die folgenden Verbindungen der Formel (I) genannt (X steht sowohl für ein Stickstoffatom als auch die CH-Gruppe) :

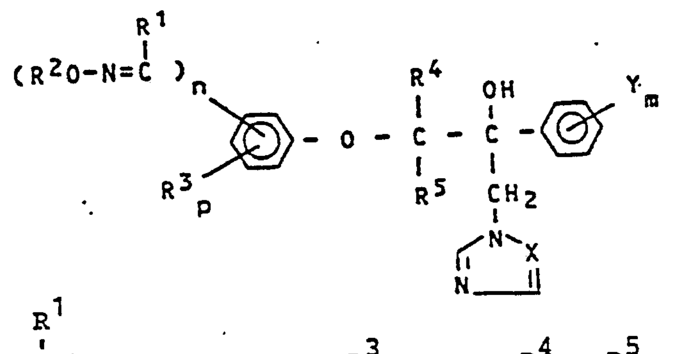

(I)

| $(R^2O-N=C(R^1)-)_n$ | $R^3_p$ | $R^4$ | $R^5$ | $Y_m$ |
|---|---|---|---|---|
| 4- $CH_3O-N=CH-$ | 2-Cl | $CH_3$ | $CH_3$ | 2-F |
| 4-$CH_3O-N=CH-$ | 2-F | $CH_3$ | $CH_3$ | 2-F |

(Fortsetzung)

| $(R^2O-N=C(R^1)-)_n$ | $R^3_p$ | $R^4$ | $R^5$ | $Y_m$ |
|---|---|---|---|---|
| 2-$CH_3O$-N=CH- | - | $CH_3$ | $CH_3$ | 2-F |
| 4-$CH_3O$-N=CH- | - | $CH_3$ | $CH_3$ | 2-F |
| 2-$CH_3O$-N=CH- | 4-Cl | $CH_3$ | $CH_3$ | 2-F |
| 2-$CH_3O$-N-CH- | 4-F | $CH_3$ | $CH_3$ | 2-F |
| 4-$C_4H_9O$-N=CH- | - | $CH_3$ | $CH_3$ | 2-F |
| 4-$CH_2$=CH-$CH_2O$-N=CH- | - | $CH_3$ | $CH_3$ | 2-F |
| 4-HC≡C-$CH_2O$-N=CH- | - | $CH_3$ | $CH_3$ | 2-F |
| 4- Cl-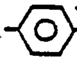-$CH_2$-O-N=CH- | - | $CH_3$ | $CH_3$ | 2-F |
| 4-$CH_3O$-N=C- 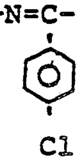 | - | $CH_3$ | $CH_3$ | 2-F |
| 4-$CH_3O$-N=C($CH_3$)- | - | $CH_3$ | $CH_3$ | 2-F |
| 2-$CH_3O$-N=C($CH_3$)- | - | $CH_3$ | $CH_3$ | 2-F |
| 4-$CH_3O$-N=CH- | 2-Cl | $CH_3$ | $CH_3$ | 4-Cl |
| 4-$CH_3O$-N=CH- | 2-F | $CH_3$ | $CH_3$ | 4-Cl |
| 2-$CH_3O$-N=CH- | - | $CH_3$ | $CH_3$ | 4-Cl |
| 2-$CH_3O$-N=CH- | 4-Cl | $CH_3$ | $CH_3$ | 4-Cl |
| 2-$CH_3O$-N=CH- | 4-F | $CH_3$ | $CH_3$ | 4-Cl |
| 4-$C_4H_9O$-N=CH- | - | $CH_3$ | $CH_3$ | 4-Cl |
| 4-$CH_2$=CH-$CH_2O$-N=CH- | - | $CH_3$ | $CH_3$ | 4-Cl |
| 4-CH≡C-$CH_2O$-N=CH- | - | $CH_3$ | $CH_3$ | 4-Cl |
| 4-Cl-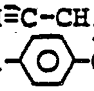-$CH_2O$-N=CH- | - | $CH_3$ | $CH_3$ | 4-Cl |
| 4-$CH_3O$-N=C- 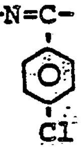 | - | $CH_3$ | $CH_3$ | 4-Cl |
| 2-$CH_3O$-N=C($CH_3$)- | - | $CH_3$ | $CH_3$ | 4-Cl |
| 4-$CH_3O$-N=CH- | 2-Cl | $CH_3$ | $CH_3$ | 2-Cl |
| 4-$CH_3O$-N=CH- | 2-F | $CH_3$ | $CH_3$ | 2-Cl |
| 2-$CH_3O$-N=C($CH_3$)- | - | $CH_3$ | $CH_3$ | 2-Cl |
| 2-$CH_3O$-N=CH- | - | $CH_3$ | $CH_3$ | 2-Cl |
| 2-$CH_3O$-N=CH- | 4-Cl | $CH_3$ | $CH_3$ | 2-Cl |
| 2-$CH_3O$-N=CH- | 4-F | $CH_3$ | $CH_3$ | 2-Cl |
| 4-$C_4H_9O$-N=CH- | - | $CH_3$ | $CH_3$ | 2-Cl |
| 4-$CH_2$=CH-$CH_2O$-N=CH- | - | $CH_3$ | $CH_3$ | 2-Cl |
| 4-CH≡C-$CH_2O$-N=CH- | - | $CH_3$ | $CH_3$ | 2-Cl |
| 4-Cl-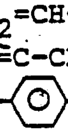-$CH_2O$-N=CH- | - | $CH_3$ | $CH_3$ | 2-Cl |

(Fortsetzung)

| $(R^2O-N=C(R^1)-)_n$ | $R^3_p$ | $R^4$ | $R^5$ | $Y_m$ |
|---|---|---|---|---|
| 4-$CH_3O$-N=C- 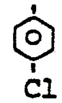 | - | $CH_3$ | $CH_3$ | 2-Cl |
| 4-$CH_3$-O-N=C($CH_3$)- | - | $CH_3$ | $CH_3$ | 2-Cl |
| 4-$CH_3O$-N=CH- | 2-Cl | $CH_3$ | $CH_3$ | 4-F |
| 4-$CH_3O$-N=CH- | 2-F | $CH_3$ | $CH_3$ | 4-F |
| 2-$CH_3O$-N=CH- | - | $CH_3$ | $CH_3$ | 4-F |
| 2-$CH_3O$-N=C($CH_3$)- | - | $CH_3$ | $CH_3$ | 4-F |
| 2-$CH_3O$-N=CH- | 4-Cl | $CH_3$ | $CH_3$ | 4-F |
| 2-$CH_3O$-N=CH- | 4-F | $CH_3$ | $CH_3$ | 4-F |
| 4-$C_4H_9O$-N=CH- | - | $CH_3$ | $CH_3$ | 4-F |
| 4-$CH_2$=CH-$CH_2O$-N=CH- | - | $CH_3$ | $CH_3$ | 4-F |
| 4-CH≡C-$CH_2O$-N=CH- | - | $CH_3$ | $CH_3$ | 4-F |
| 4-Cl-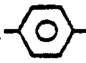-$CH_2O$-N=CH- | - | $CH_3$ | $CH_3$ | 4-F |
| 4-$CH_3O$-N=C- 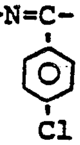 | - | $CH_3$ | $CH_3$ | 4-F |
| 4-$CH_3O$-N=C($CH_3$)- | - | $CH_3$ | $CH_3$ | 4-F |
| 4-$CH_3O$-N=CH- | 2-Cl | $CH_3$ | $CH_3$ | 2,4-$Cl_2$ |
| 4-$CH_3O$-N=CH- | 2-F | $CH_3$ | $CH_3$ | 2,4-$Cl_2$ |
| 2-$CH_3O$-N=CH-- | - | $CH_3$ | $CH_3$ | 2,4-$Cl_2$ |
| 4-$CH_3O$-N=CH- | - | $CH_3$ | $CH_3$ | 2,4-$Cl_2$ |
| 2-$CH_3O$-N=CH- | 4-Cl | $CH_3$ | $CH_3$ | 2,4-$Cl_2$ |
| 2-$CH_3O$-N=CH- | 4-F | $CH_3$ | $CH_3$ | 2,4-$Cl_2$ |
| 4-$C_4H_9$-N=CH- | - | $CH_3$ | $CH_3$ | 2,4-$Cl_2$ |
| 4-$CH_2$=CH-$CH_2O$-N=CH- | - | $CH_3$ | $CH_3$ | 2,4-$Cl_2$ |
| 4-CH≡C-$CH_2O$-N=CH- | - | $CH_3$ | $CH_3$ | 2,4-$Cl_2$ |
| 4-Cl-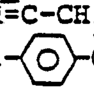-$CH_2O$-N=CH- | - | $CH_3$ | $CH_3$ | 2,4-$Cl_2$ |
| 4-$CH_3O$-N=C- 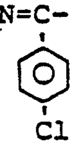 | - | $CH_3$ | $CH_3$ | 2,4-$Cl_2$ |
| 4-$CH_3O$-N=C($CH_3$)- | - | $CH_3$ | $CH_3$ | 2,4-$Cl_2$ |
| 2-$CH_3O$-N=C($CH_3$)- | - | $CH_3$ | $CH_3$ | 2,4-$Cl_2$ |
| 4-$CH_3O$-N=CH- | - | $CH_3$ | $CH_3$ | 3-Cl |
| 4-$CH_3O$-N=CH- | - | $CH_3$ | $CH_3$ | 3-F |
| 4-$CH_3O$-N=CH- | - | $CH_3$ | $CH_3$ | 4-Br |

(Fortsetzung)

| $(R^2O-N=\overset{R^1}{C}-)_n$ | $R^3_p$ | $R^4$ | $R^5$ | $Y_m$ |
|---|---|---|---|---|
| $4-CH_3O-N=CH-$ | - | $CH_3$ | $CH_3$ | $2,5-Cl_2$ |
| $4-CH_3O-N=CH-$ | - | $CH_3$ | $CH_3$ | $4-CH_3$ |
| $4-CH_3O-N=CH-$ | - | $CH_3$ | $CH_3$ | $2-CH_3$ |
| $4-CH_3O-N=CH-$ | - | $CH_3$ | $CH_3$ | $4-OCF_3$ |
| $4-CH_3O-N=CH-$ | - | $CH_3$ | $CH_3$ | $4-SCF_3$ |
| $4-CH_3O-N=CH-$ | - | $CH_3$ | $CH_3$ | $4-CF_3$ |

Verwendet man beispielsweise 2-(4-Chlorphenyl)-2-[2(4-methoximinomethylphenoxy)-prop-2-yl]-oxiran und 1,2,4-Triazol als Ausgangsstoffe, so kann der Ablauf des erfindungsgemäßen Verfahrens durch das folgende Formelschema wiedergegebenen werden :

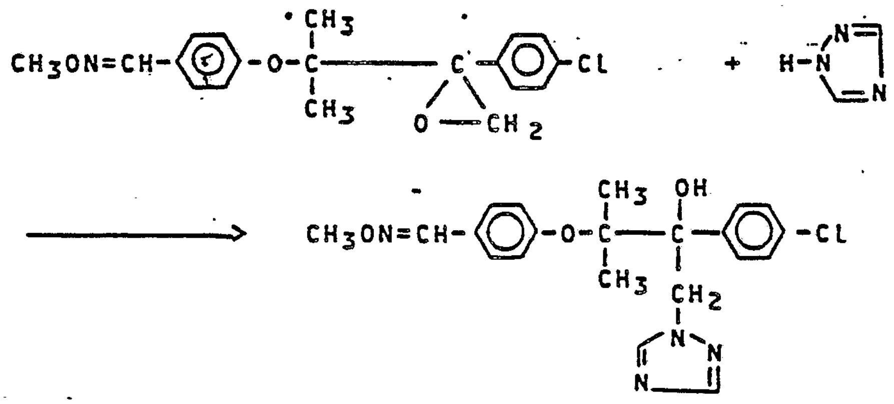

Die für die Durchführung des erfindungsgemäßen Verfahrens als Ausgangsstoffe zu verwendenden Oxirane sind durch die Formel (II) allgemein definiert. In dieser Formel haben $R^1$, $R^2$, $R^3$, $R^4$, $R^5$ und Y sowie die Indizes m, n und p vorzugsweise die Bedeutungen, die bereits im Zusammenhang mit der Beschreibung der erfindungsgemäßen Stoffe der Formel (I) vorzugsweise für diese Substituenten bzw. Indizes genannt wurden.

Die Oxirane der Formel (II) sind noch nicht bekannt. Sie können jedoch in allgemein bekannter Art und Weise erhalten werden, indem man Ketone der Formel

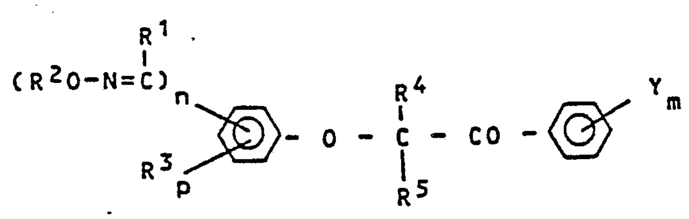

(IV)

in welcher $R^1$, $R^2$, $R^3$, $R^4$, $R^5$, Y und die Indizes m, n und p die oben angegebene Bedeutung haben, entweder

α) mit Dimethylsulfonium-methylid der Formel

$$\overset{\delta+\delta-}{(CH_3)_2SOCH_2} \qquad (V)$$

in Gegenwart eines Verdünnungsmittels umsetzt, oder

β) mit Trimethylsulfonium-methylsulfat der Formel

$$[(CH_3)_3S^+]\ CH_3SO_4^- \qquad (VI)$$

in Gegenwart eines inerten organischen Lösungsmittels sowie in Gegenwart einer Base umsetzt.

Das bei der Verfahrensvariante (α) benötigte Dimethyloxosulfoniummethylid der Formel (V) ist bekannt (vergleiche J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (VI) ist ebenfalls bekannt (vgl. Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (α) des Verfahrens zur Herstellung der Oxirane der Formel (II) kommt als Verdünnungsmittel vorzugsweise Dimethylsulfoxid infrage.

Die Reaktionstemperaturen können bei der oben beschriebenen Verfahrensvariante (α) innerhalb eines größeren Bereiches variiert werden. Im allgemeinen arbeitet man bei Temperaturen zwischen 20 °C und 80 °C.

Die Durchführung des Verfahrens zur Herstellung der Oxirane der Formel (II) nach der Variante (α) und die Aufarbeitung des bei dieser Synthese anfallenden Reaktionsgemisches erfolgen nach üblichen Methoden (vgl. J. Am. Chem. Soc. 87, 1363-1364 (1965)).

Bei der Variante (β) zur Herstellung der Oxirane der Formel (II) kommt als inertes organisches Lösungsmittel vorzugsweise Acetonitril in Betracht.

Als Basen können bei der Verfahrensvariante (β) starke anorganische oder organische Basen verwendet werden. Vorzugsweise infrage kommt Natriummethylat.

Die reaktionstemperaturen können bei der oben beschriebenen Verfahrensvariante (β) innerhalb eines bestimmten Bereiches variiert werden. Im allgemeinen arbeitet man bei Temperaturen zwischen 0 °C und 60 °C, vorzugsweise bei Raumtemperatur.

Die Durchführung des Verfahrens zur Herstellung der Oxirane der Formel (II) nach der Variante (β) und die Aufarbeitung des bei dieser Synthese anfallenden Reaktionsproduktes erfolgen nach üblichen Methoden (vgl. Heterocycles 8, 397 (1977)).

Die Oxirane der Formel (II) können bei dem erfindungsgemäßen Verfahren gegebenenfalls ohne Isolierung direkt weiter umgesetzt werden.

Die bei der Herstellung der Oxirane der Formel (II) als Ausgangsstoffe benötigten Ketone der Formel (IV) werden erhalten, indem man entsprechende Halogenketone der Formel

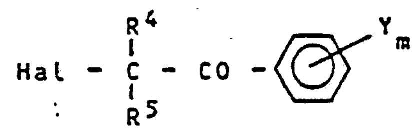

(VII)

in welcher $R^4$, $R^5$, Y und der Index n die oben angegebene Bedeutung haben und Hal für Halogen, vorzugsweise Chlor oder Brom, steht, mit Phenolen der Formel

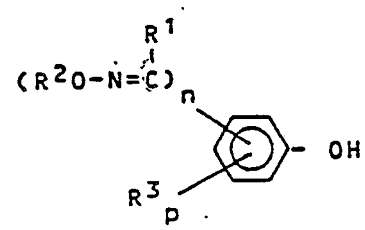

(VIII)

in welcher $R^1$, $R^2$, $R^3$ und die Indizes n und p die oben angegebene Bedeutung haben, in Gegenwart eines inerten organischen Lösungsmittels, wie beispielsweise Toluol, und in Gegenwart eines Säurebindemittels, wie beispielsweise Kaliumcarbonat, bei Temperaturen zwischen 80 und 120 °C erhitzt (vgl. auch die Herstellungsbeispiele).

Die Halogenketone der Formel (VIII) und die Phenole der Formel (VIII) sind bekannt, bzw. können sie in bekannter Art und Weise erhalten werden.

Die außerdem für das erfindungsgemäße Verfahren als Ausgangsstoffe zu verwendenden Azole sind durch die Formel (III) allgemein definiert. In dieser Formel steht X vorzugsweise für die Bedeutungen, die bereits in der Erfindungsdefinition für diesen Substituenten genannt wurden. M steht vorzugsweise für Wasserstoff, Natrium oder Kalium.

Als Verdünnungsmittel kommen für das erfindungsgemäße Verfahren unter den Reaktionsbedingungen inerte organische Lösungsmittel infrage. Hierzu gehören vorzugsweise Alkohole, wie z. B. Ethanol, Methoxyethanol oder Propanol ; Ketone, wie z. B. 2-Butanon, Nitrile, wie z. B. Acetonitril ; Ester, wie Essigester ; Ether, wie z. B. Dioxan ; aromatische Kohlenwasserstoffe, wie z. B. Benzol und Toluol ; oder Amide, wie z. B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z. B. Natrium- und Kaliumcarbonat ; Alkalihydroxide, wie z. B. Natriumhydroxid ; Alkalialkoholate, wie z. B. Natrium- und Kalium-methylat und -ethylat ; Alkalihydride, wie z. B. Natriumhydrid ; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0 und 200 °C, vorzugsweise zwischen 60 und 150 °C.

Bei der Durchführung des erfindungsgemäßen Verfahrens setzt man vorzugsweise auf 1 Mol Oxiran der Formel (II) 1 bis 2 Mol Azol der Formel (III) und gegebenenfalls 1 bis 2 Mol Base ein ; die Isolierung der Endprodukte erfolgt in allgemein üblicher Weise.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I), deren Ester-Derivate und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bi-phasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulospsis-Arten, wie Torulospsis glabrata. Die Aufzählung dieser Microorganismen stellt keinsfalls eine Beschränkung der bekämpfbaren Keime dar, sondern hat nur erläuternden Charakter.

Als Indikationsbeispiele in der Humanmedizin können beispielsweise genannt werden :

Dermatomykosen und Systemmykosen durch Trichophyton mentagrophytes und andere Trichophytonarten, Microsporonarten, Epidermophyton floccosum, Sproßpilze und biphasische Pilze sowie Schimmelpilze hervorgerufen.

Als Indikationsgebiete in der Tiermedizin können beispielsweise aufgeführt werden :

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Mengen Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen, seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaloin und Bentonit und (i) Gleitmittel, z. B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teils des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Ethylalkohol, Propylalkohol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

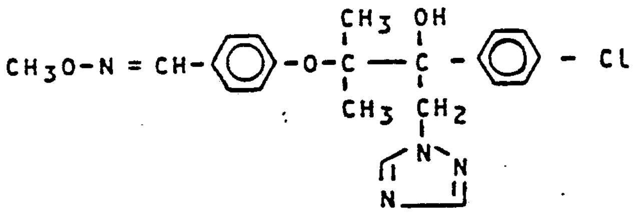

Zu einer Lösung von 12,2 g (0,176 Mol) 1,2,4-Triazol und 0,35 g (0,0153 Mol) Natrium in 100 ml n-Propanol werden bei 90 °C 52,8 g (0,153 Mol) 2-(4-Chlorphenyl)-2-[2-(4-methoximinomethylphenoxy)-prop-2-yl]-oxiran in 60 ml n-Propanol getropft. Man läßt das Reaktionsgemisch 20 Stunden bei 90 °C nachrühren, kühlt ab und engt im Vakuum ein. Der Rückstand wird säulenchromatographisch (Kieselgel ; Essigester/Cyclohexan = 3/1) gereinigt. Man erhält 10,3 g (16,3 % der Theorie) 2-(4-methoximino-methylphenoxy)-3-methyl-1-(1,2,4-triazol-1-yl)-2-butanol vom Schmelzpunkt 54 °C.

Herstellung des Ausgangsproduktes

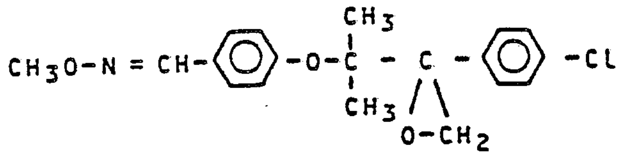

Eine Lösung von 40,8 g (0,320 Mol) Dimethylsulfat und 21,8 g (0,355 Mol) Dimethylsulfid in 190 ml Acetonitril läßt man 5 Tage bei Raumtemperatur rühren. Man versetzt anschließend mit 59,9 g (0,181 Mol) 4-Chlorphenyl-[2-(4-methoximino-methyl-phenoxy)-2-propyl]-keton sowie 19,7 g Natriummethylat, läßt das Reaktionsgemisch 20 Stunden bei Raumtemperatur nachrühren und engt anschließend durch Destillation im Vakuum ein. Der Rückstand wird mit einem Gemisch aus 140 ml Essigester und 110 ml Wasser verrührt.

Die organische Phase wird abgetrennt, über Natriumsulfat getrocknet und im Vakuum eingeengt. Man erhält 52,8 g (84,6 % der Theorie) rohes 2-(4-Chlorphenyl)-2-[2-(4-methoximino-methyl-phenoxy)-prop-2-yl]-oxiran als zähes Öl, das ohne Reinigung direkt weiter umgesetzt wird.

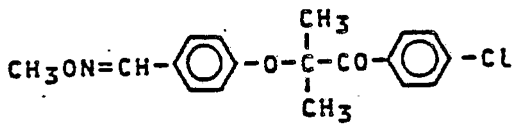

50 g (0,331 Mol) 4-Methoximino-methyl-phenol und 45,7 g (0,331 Mol) Kaliumcarbonat in 200 Toluol werden 1 Stunde unter Rückfluß am Wasserabscheider erhitzt. Man kühlt auf ca. 40 °C ab und versetzt tropfenweise mit 69,8 g 2-Brom-prop-2-yl-(4-chlorphenyl)-Keton. Dieses Reaktionsgemisch läßt man 5 Stunden bei 100 °C nachrühren und saugt dann über eine Nutsche ab. Das Filtrat wird dreimal mit 10 %-iger Natronlauge gewaschen, mit Wasser nachgewaschen, über Natriumsulfat getrocknet und im Vakuum eingeengt. Man erhält 59,9 g rohes (67,7 % der Theorie) 4-Chlorphenyl-[2-(4-methoximino-methyl-phenoxy)-2-propyl]-keton als dunkles Öl, das ohne Reinigung direkt umgesetzt wird.

In entsprechender Weise und gemäß dem erfindungsgemäßen Verfahren werden die folgenden Verbindungen der allgemeinen Formel

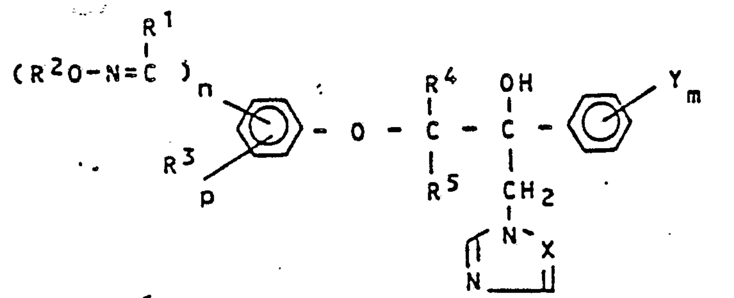

(I)

erhalten :

| Bsp. Nr. | 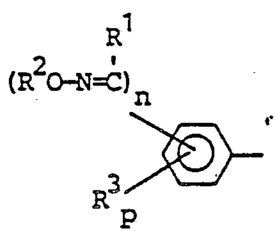 | $R^4$ | $R^5$ | X | $Y_m$ | Schmelzpunkt (°C) bzw. $n_D^{20}$ |
|---|---|---|---|---|---|---|
| 2 | 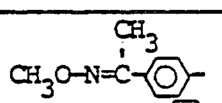 | $CH_3$ | $CH_3$ | N | 4-Cl | 52 |
| 3 | 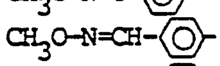 | $CH_3$ | $CH_3$ | N | 4-F | 35 |
| 4 | 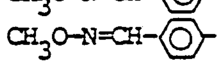 | $CH_3$ | $CH_3$ | N | 2-Cl | |
| 5 | 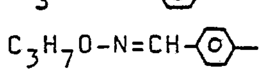 | $CH_3$ | $CH_3$ | N | 4-Cl | Oel |

Verwendungsbeispiele

In dem nachfolgenden Beispiel werden die nachstehend angegebenen Verbindungen als Vergleichssubstanzen eingesetzt :

(A) 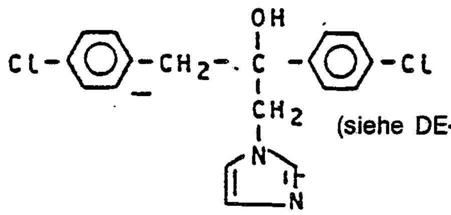 (siehe DE-OS 2 623 129)

(B) 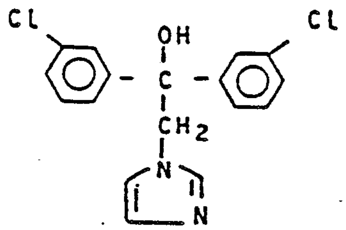 (siehe DE-OS 2 623 129)

(C) 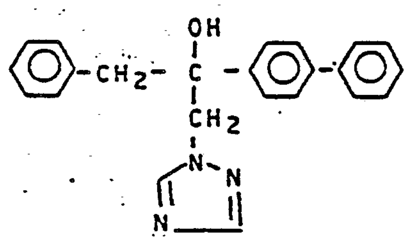 (siehe DE-OS 2 851 086)

Beispiel A

Antimykotische in-vivo-Wirksamkeit (oral) bei Mäusecandidose

Versuchsbeschreibung

Mäuse vom Typ SPF-$CF_1$ wurden intravenös mit 1-2 × $10^6$ logarithmisch wachsenden Candida-Zellen, die in physiologischen Kochsalzlösung suspendiert werden, infiziert. Eine Stunde vor und sieben Stunden nach der Infektion werden die Tiere mit jeweils 25-100 mg/kg Körpergewicht der Präparate oral behandelt.

Ergebnis

Unbehandelte Tiere starben 3 bis 6 Tage post infektionem.

Die Überlebensrate am 6. Tag post infektionem betrug bei unbehandelten Kontrolltieren etwa 5 %.

In diesem Test zeigen z. B. die erfindungsgemäßen Verbindungen 1 und 2 eine bessere Wirkung als die aus dem Stand der Technik bekannten Verbindungen (A), (B) und (C).

Zeichenerklärung

+++++ = sehr gut Wirkung = 90 % Überlebende am 6. Tag p.i.
++++ = gute Wirkung = 80 % Überlebende am 6. Tag p.i.
+++ = Wirkung = 60 % Überlebende am 6. Tag p.i.
++ = schwache Wirkung = 40 % Überlebende am 6. Tag p.i.
+ = Spur Wirkung
k. W. = keine Wirkung

Tabelle A

Antimykotische in-vivo-Wirksamkeit (oral) bei Mäusecandidose

| Wirkstoff | Wirkung |
|---|---|
| (A) (bekannt) | + |
| (B) (bekannt) | k. W. |
| (C) (bekannt) | k. W. |
| Verbindungen gemäß Herst. Bsp. | |
| 1 | +++++ |
| 2 | ++++ |

Beispiel B/Formulierungen

1) Lösung

| | |
|---|---|
| Wirkstoff gemäß Formel (I): | 10 g |
| Alkohol, rein (96 %-ig): | 300 g |
| Isopropylmyristat: | 526 g |
| | 836 g |

2) Creme

| | |
|---|---|
| Wirkstoff gemäß Formel (I): | 10 g |
| Aralcel 60: (Sorbitan-monostearat) | 20 g |
| Tween 60: (Polyoxyethylen(20)-sorbitan-monostearat) | 15 g |
| Walrat, künstlich: (Mischung vom Estern von gesättigten Fettsäuren $C_{14}$-$C_{18}$ und Fettalkoholen $C_{14}$-$C_{18}$ | 30 g |
| Lanette O: (Gemisch aus Cetyl-Alkohol und Stearyl-Alkohol) | 100 g |
| Entanol G: (2-Octyl-dodecanol) | 135 g |
| Benzylalkohol: | 10 g |
| Wasser, entmineralisiert: | 680 g |
| | 1 000 g |

**Patentansprüche**

1. Substituierte Hydroxyalkyl-azole der allgemeinen Formel

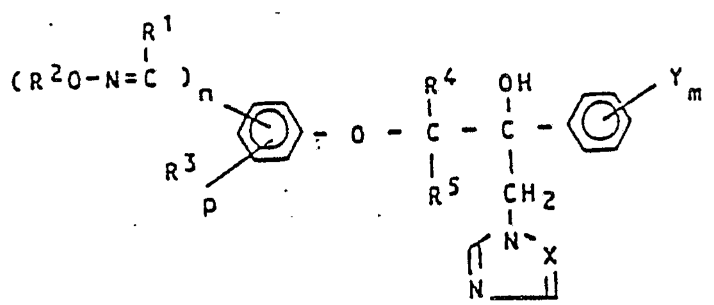

(I)

in welcher

$R^1$ für Wasserstoff, geradkettiges oder verzweigtes Alkyl mit 1 bis 10 Kohlenstoffatomen sowie für jeweils gegebenenfalls einfach bis dreifach, gleich oder verschieden substituiertes Phenyl oder Benzyl, wobei als Substituenten die Bedeutungen von Y infrage kommen;

$R^2$ für Wasserstoff, geradkettiges oder verzweigtes Alkyl mit 1 bis 10 Kohlenstoffatomen, geradkettiges oder verzweigtes Alkenyl und Alkinyl mit jeweils 3 bis 10 Kohlenstoffatomen sowie für jeweils gegebenenfalls einfach bis dreifach, gleich oder verschieden substituiertes Phenyl und Benzyl, wobei als Substituenten die Bedeutungen von Y infrage kommen;

n für die Zahlen 1 oder 2;

$R^3$ für die Bedeutungen von Y;

p für die Zahlen 0, 1 oder 2 ;
$R^4$ für geradkettiges oder verzweigtes Alkyl mit 1 bis 4 Kohlenstoffatomen ;
$R^5$ für geradkettiges oder verzweigtes Alkyl mit 1 bis 4 Kohlenstoffatomen ;
X für ein Stickstoffatom oder die CH-Gruppe ;
Y für Halogen, Alkyl mit 1 bis 4 Kohlenstoffatomen, Cycloalkyl mit 5-7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, und
m für die Zahlen 0, 1, 2 oder 3
stehen.

2. Substituierte Hydroxyalkyl-azole der allgemeinen Formel (I) in Anspruch 1, in der
$R^1$ für Wasserstoff, geradkettiges oder verzweigtes Alkyl mit 1 bis 4 Kohlenstoffatomen sowie für jeweils gegebenenfalls einfach oder zweifach, gleich oder verschieden substituiertes Phenyl und Benzyl steht, wobei als Substituenten die Bedeutungen von Y infrage kommen ;
$R^2$ für Wasserstoff, geradkettiges oder verzweigtes Alkyl mit 1 bis 4 Kohlenstoffatomen, geradkettiges oder verzweigtes Alkenyl und Alkinyl mit jeweils 3 bis 4 Kohlenstoffatomen sowie für jeweils gegebenenfalls einfach oder zweifach, gleich oder verschieden substituiertes Phenyl und Benzyl steht, wobei als Substituenten die Bedeutungen von Y infrage kommen ;
n für die Zahlen 1 oder 2 steht ;
$R^3$ für die Bedeutungen von Y steht ;
p für die Zahlen 0, 1 oder 2 steht ;
$R^4$ für Methyl oder Ethyl steht ;
$R^5$ für Methyl oder Ethyl steht ;
X für ein Stickstoffatom oder die CH-Gruppe steht ;
Y für Fluor, Chlor, Brom, Methyl, Isopropyl, tert.-Butyl, Cyclohexyl, Methoxy, Methylthio, Trifluormethyl, Trifluormethoxy, Trifluormethylthio,
und
m für die Zahlen 0, 1 oder 2 steht.

3. 2-(4-Chlorphenyl)-3-(4-methoximino-methyl-phenoxy)-3-methyl-1-(1,2,4-triazol-1-yl)-2-butanol.

4. 2-(4-Chlorphenyl)-3-[4-(1-methoximino-1-ethyl)-phenoxy]-3-methyl-1-(1,2,4-triazol-1-yl)-2-butanol.

5. Substituierte Hydroxyalkyl-azole der allgemeinen Formel

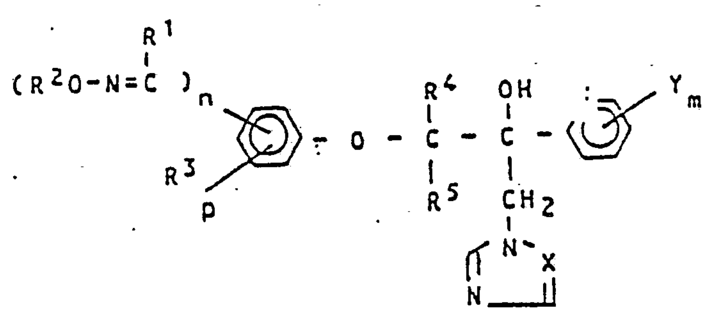

(I)

in welcher
$R^1$ für Wasserstoff, geradkettiges oder verzweigtes Alkyl mit 1 bis 10 Kohlenstoffatomen sowie für jeweils gegebenenfalls einfach bis dreifach, gleich oder verschieden substituiertes Phenyl oder Benzyl, wobei als Substituenten die Bedeutungen von Y infrage kommen ;
$R^2$ für Wasserstoff, geradkettiges oder verzweigtes Alkyl mit 1 bis 10 Kohlenstoffatomen, geradkettiges oder verzweigtes Alkenyl und Alkinyl mit jeweils 3 bis 10 Kohlenstoffatomen sowie für jeweils gegebenenfalls einfach bis dreifach, gleich oder verschieden substituiertes Phenyl und Benzyl, wobei als Substituenten die Bedeutungen von Y infrage kommen ;
n für die Zahlen 1 oder 2 ;
$R^3$ für die Bedeutungen von Y ;
p für die Zahlen 0, 1 oder 2 ;
$R^4$ für geradkettiges oder verzweigtes Alkyl mit 1 bis 4 Kohlenstoffatomen ;
$R^5$ für geradkettiges oder verzweigtes Alkyl mit 1 bis 4 Kohlenstoffatomen ;
X für ein Stickstoffatom oder die CH-Gruppe ;
Y für Halogen, Alkyl mit 1 bis 4 Kohlenstoffatomen, Cycloalkyl mit 5-7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, und
m für die Zahlen 0, 1, 2 oder 3
stehen, sowie deren Säureadditionssalze bei der Behandlung von Mykosen.

6. Verwendung von substituierten Hydroxyalk-azolen der allgemeinen Formel

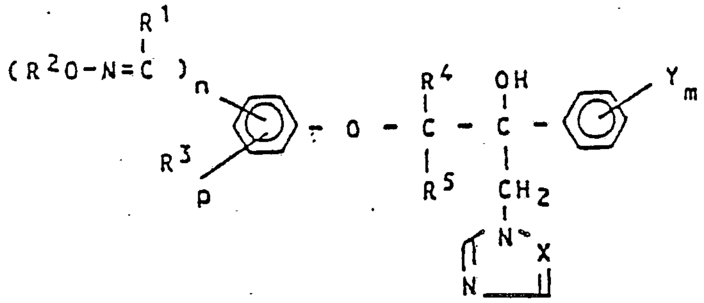

(I)

in welcher

$R^1$ für Wasserstoff, geradkettiges oder verzweigtes Alkyl mit 1 bis 10 Kohlenstoffatomen sowie für jeweils gegebenenfalls einfach bis dreifach, gleich oder verschieden substituiertes Phenyl oder Benzyl, wobei als Substituenten die Bedeutungen von Y infrage kommen ;

$R^2$ für Wasserstoff, geradkettiges oder verzweigtes Alkyl mit 1 bis 10 Kohlenstoffatomen, geradkettiges oder verzweigtes Alkenyl und Alkinyl mit jeweils 3 bis 10 Kohlenstoffatomen sowie für jeweils gegebenenfalls einfach bis dreifach, gleich oder verschieden substituiertes Phenyl und Benzyl, wobei als Substituenten die Bedeutungen von Y infrage kommen ;

n für die Zahlen 1 oder 2 ;

$R^3$ für die Bedeutungen von Y ;

p für die Zahlen 0, 1 oder 2 ;

$R^4$ für geradkettiges oder verzweigtes Alkyl mit 1 bis 4 Kohlenstoffatomen ;

$R^5$ für geradkettiges oder verzweigtes Alkyl mit 1 bis 4 Kohlenstoffatomen ;

X für ein Stickstoffatom oder die CH-Gruppe ;

Y für Halogen, Alkyl mit 1 bis 4 Kohlenstoffatomen, Cycloalkyl mit 5-7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleiche oder verschiedenen Halogenatomen, wie vorzugsweise Fluor- und Chloratome, und

m für die Zahlen 0, 1, 2 oder 3

stehen, sowie deren Säureadditionssalze bei der Zubereitung von Arzneimitteln zur Behandlung von Mykosen.

7. Verfahren zur Herstellung von substituierten Hydroxyalkyl-azolen der allgemeinen Formel (I) in Anspruch 1, dadurch gekennzeichnet, daß man Oxirane der Formel

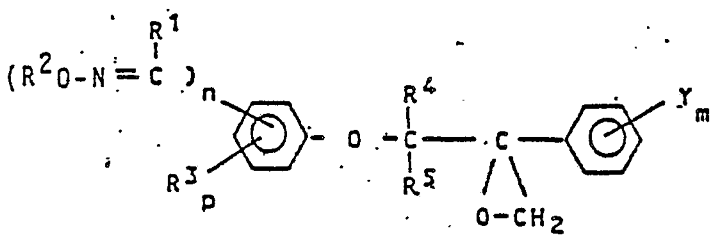

(II)

in welcher $R^1$, $R^2$, $R^3$, $R^4$, $R^5$, Y und die Indizes m, n und p die in Anspruch 1 angegebene Bedeutung haben, mit Azolen der Formel

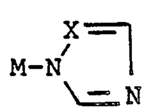

(III)

in welcher

X die in Anspruch 1 angegebene Bedeutung hat und

M für Wasserstoff oder ein Alkalimetall steht,

in Gegenwart eines Verdünnungsmittels und gegebenenfalls in Gegenwart einer Base umsetzt und an die so erhaltenen Verbindungen gegebenenfalls eine Säure addiert.

8. Arzneimittel enthaltend substituierte Hydroxyalkylazole der allgemeinen Formel (I) in Anspruch 1.

9. Verfahren zur Herstellung eines Arzneimittels, dadurch gekennzeichnet, daß man Hydroxyalkylazole der allgemeinen Formel (I) in Anspruch 1 mit inerten, nichttoxischen, pharmazeutisch geeigneten trägerstoffen vermischt.

**Claims**

1. Substituted hydroxyalkyl-azoles of the general formula

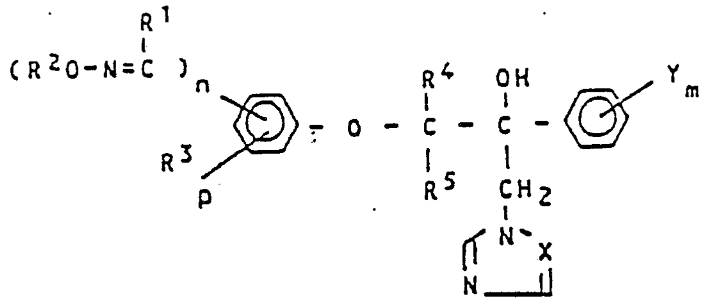

(I)

in which

$R^1$ represents hydrogen or straight-chain or branched alkyl with 1 to 10 carbon atoms, or represents phenyl or benzyl, each of which is optionally mono-, di- or tri-substituted by identical or different substituents, possible substituents having the meanings of Y ;

$R^2$ represents hydrogen, straight-chain or branched alkyl with 1 to 10 carbon atoms or straight-chain or branched alkenyl or alkinyl with in each case 3 to 10 carbon atoms, or represents phenyl or benzyl, each of which is optionally mono-, di- or tri-substituted by identical or different substituents, possible substituents having the meanings of Y ;

n represents the number 1 or 2 ;

$R^3$ represents the meanings of Y ;

p represents the number 0, 1 or 2 ;

$R^4$ represents straight-chain or branched alkyl with 1 to 4 carbon atoms ;

$R^5$ represents straight-chain or branched alkyl with 1 to 4 carbon atoms ;

X represents a nitrogen atom or the CH group ;

Y represents halogen, alkyl with 1 to 4 carbon atoms, cycloalkyl with 5-7 carbon atoms, alkoxy or alkylthio with in each case 1 to 4 carbon atoms or halogenoalkyl, halogenoalkoxy or halogenoalkylthio with in each 1 or 2 carbon atoms and 1 to 5 identical or different halogen atoms, such as, preferably, fluorine and chlorine atoms, and

m represents the number 0, 1, 2 or 3.

2. Substituted hydroxyalkyl-azoles of the general formula (I) in Claim 1, in which

$R^1$ represents hydrogen or straight-chain or branched alkyl with 1 to 4 carbon atoms, or represents phenyl or benzyl, each of which is optionally mono- or di-substituted by identical or different substituents, possible substituents having the meanings of Y ;

$R^2$ represents hydrogen, straight-chain or branched alkyl with 1 to 4 carbon atoms or straight-chain or branched alkenyl or alkinyl with in each case 3 or 4 carbon atoms, or represents phenyl or benzyl, in each case optionally mono- or di-substituted by identical or different substituents, possible substituents having the meanings of Y ;

n represents the number 1 or 2 ;

$R^3$ represents the meanings of Y ;

p represents the number 0, 1 or 2 ;

$R^4$ represents methyl or ethyl ;

$R^5$ represents methyl or ethyl ;

X represents a nitrogen atom or the CH group ;

Y represents fluorine, chlorine, bromine, methyl, isopropyl, tert.-butyl, cyclohexyl, methoxy, methylthio, trifluoromethyl, trifluoromethoxy or trifluoromethylthio, and

m represents the number 0, 1 or 2.

3. 2-(4-Chlorophenyl)-3-(4-methoximino-methyl-phenoxy)-3-methyl-1-(1,2,4-triazol-1-yl)-2-butanol.

4. 2-(4-chlorophenyl)-3-[4-(1-methoximino-1-ethyl)-phenoxy]-3-methyl-1-(1,2,4-triazol-1-yl)-2-butanol.

5. Substituted hydroxyalkyl-azoles of the general formula

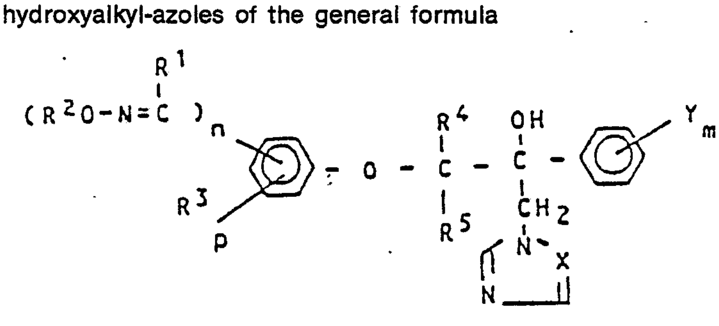

(I)

in which

$R^1$ represents hydrogen or straight-chain or branched alkyl with 1 to 10 carbon atoms, or represents phenyl or benzyl, each of which is optionally mono-, or tri-substituted by identical or different substituents, possible substituents having the meanings of Y ;

$R^2$ represents hydrogen, straight-chain or branched alkyl with 1 to 10 carbon atoms or straight-chain or branched alkenyl or alkinyl with in each case 3 to 10 carbon atoms, or represents phenyl or benzyl, each of which is optionally mono-, di- or tri-substituted by identical or different substituents, possible substituents having the meanings of Y ;

n represents the number 1 or 2 ;

$R^3$ represents the meanings of Y ;

p represents the number 0, 1 or 2 ;

$R^4$ represents straight-chain or branched alkyl with 1 to 4 carbon atoms ;

$R^5$ represents straight-chain or branched alkyl with 1 to 4 carbon atoms ;

X represents a nitrogen atom or the CH group ;

Y represents halogen, alkyl with 1 to 4 carbon atoms, cycloalkyl with 5-7 carbon atoms, alkoxy or alkylthio with in each case 1 to 4 carbon atoms or halogenoalkyl, halogenoalkoxy or halogenoalkylthio with in each case 1 or 2 carbon atoms and 1 to 5 identical or different halogen atoms, such as, preferably, fluorine and chlorine atoms, and

m represents the number 0, 1, 2 or 3.

and acid addition salts thereof, for the treatment of mycoses.

6. Use of substituted hydroxyalkyl-azoles of the general formula

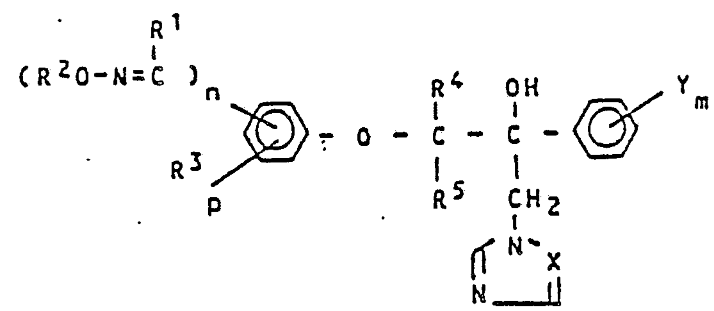

(I)

in which

$R^1$ represents hydrogen or straight-chain or branched alkyl with 1 to 10 carbon atoms, or represents phenyl or benzyl, each of which is optionally mono-, di- or tri-substituted by identical or different substituents, possible substituents having the meanings of Y ;

$R^2$ represents hydrogen, straight-chain or branched alkyl with 1 to 10 carbon atoms or straight-chain or branched alkenyl or alkinyl with in each case 3 to 10 carbon atoms, or represents phenyl or benzyl, each of which is optionally mono-, di- or tri-substituted by identical or different substituents, possible substituents having the meanings of Y ;

n represents the number 1 or 2 ;

$R^3$ represents the meanings of Y ;

p represents the number 0, 1 or 2 ;

$R^4$ represents straight-chain or branched alkyl with 1 to 4 carbon atoms ;

$R^5$ represents straight-chain or branched alkyl with 1 to 4 carbon atoms ;

X represents a nitrogen atom or the CH group ;

Y represents halogen, alkyl with 1 to 4 carbon atoms, cycloalkyl with 5-7 carbon atoms, alkoxy or alkylthio with in each case 1 to 4 carbon atoms or halogenoalkyl, halogenoalkoxy or halogenoalkylthio with in each case 1 or 2 carbon atoms and 1 to 5 identical or different halogen atoms, such as, preferably, fluorine and chlorine atoms, and

m represents the number 0, 1, 2 or 3.

and acid addition salts thereof, in the formulation of medicaments for the treatment of mycoses.

7. Process for the preparation of substituted hydroxyalkyl-azoles of the general formula (I) in Claim 1, characterized in that oxiranes of the formula

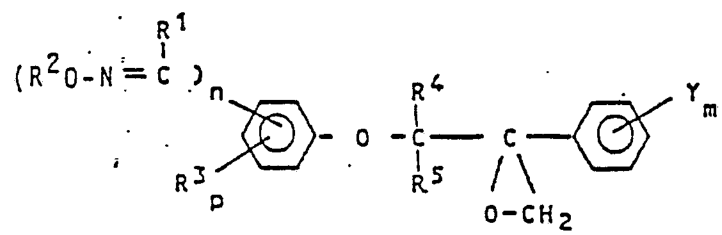

(II)

in which $R^1$, $R^2$, $R^3$, $R^4$, $R^5$, Y and the indices m, n and p have the meaning given in Claim 1, are reacted with azoles of the formula

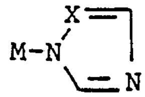

(III)

in which

X has the meaning given in Claim 1 and

M represents hydrogen or an alkali metal, in the presence of a diluent and if appropriate in the presence of a base and, if appropriate, an acid in added to the compounds thus obtained.

8. Medicaments containing substituted hydroxyalkylazoles of the general formula (I) in Claim 1.

9. Process for the preparation of a medicament, characterized in that hydroxyalkyl-azoles of the general formula (I) in Claim 1 are mixed with inert, non-toxic, pharmaceutically suitable excipients.

**Revendications**

1. Hydroxyalcoyl-azols substitués de formule générale (I)

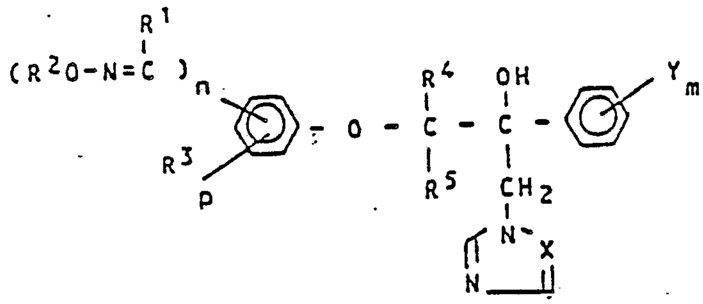

(I)

dans laquelle

$R^1$ représente de l'hydrogène, un alcoyle à chaîne droite ou ramifiée ayant 1 à 10 atomes de carbone de même qu'un phényle ou benzyle éventuellement substitués chacun une à trois fois, de manière identique ou différente, en envisageant comme substituants les significations de Y ;

$R^2$ représente de l'hydrogène, un alcoyle à chaîne droite ou ramifiée ayant 1 à 10 atomes de carbone, un alcényle et alcinyle à chaîne droite ou ramifiée ayant chacun 3 à 10 atomes de carbone de même qu'un phényle et benzyle éventuellement substitués chacun une à trois fois, de manière identique ou différente, en envisageant comme substituants les significations de Y ;

n représente les nombres 1 ou 2 ;

$R^3$ a les significations de Y ;

p signifie les nombres 0, 1 ou 2 ;

$R^4$ représente un alcoyle à chaîne droite ou ramifiée ayant 1 à 4 atomes de carbone ;

$R^5$ représente un alcoyle à chaîne droite ou ramifiée ayant 1 à 4 atomes de carbone ;

X représente un atome d'azote ou le groupe CH ;

Y représente de l'halogène, un alcoyle ayant 1 à 4 atomes de carbone, un cycloalcoyle ayant 5 à 7 atomes de carbone, un alcoxy et alcoylthio ayant chacun 1 à 4 atomes de carbone, un halogénoalcoyle et halogénoalcoxy de même que halogénoalcoylthio ayant chacun 1 à 2 atomes de carbone et 1 à 5 atomes d'halogène identiques ou différents, de préférence des atomes de fluor et de chore et

m représente les nombres 0, 1, 2 ou 3.

2. Hydroxyalcoyl-azols substitués de formule générale (I) selon la revendication 1, dans laquelle

$R^1$ représente de l'hydrogène, un alcoyle à chaîne droite ou ramifiée ayant 1 à 4 atomes de carbone, de même qu'un phényle et benzyle éventuellement substitués chacun une ou deux fois, de manière identique ou différente, en envisageant comme substituants les significations de Y ;

$R^2$ représente de l'hydrogène, un alcoyle à chaîne droite ou ramifiée ayant 1 à 4 atomes de carbone, un alcényle et alcinyle à chaîne droite ou ramifiée ayant chacun 3 à 4 atomes de carbone de même qu'une phényle et benzyle éventuellement substitués chacun une ou deux fois, de manière identique ou différente, en envisageant comme substituants les significations de Y ;

n représente les nombres 1 et 2 ;

$R^3$ a les significations de Y ;

p signifie les nombres 0, 1 ou 2 ;

$R^4$ représente un méthyle ou éthyle ;

$R^5$ un méthyle ou éthyle ;

X représente un atome d'azote ou le groupe CH ;

Y représente du fluor, chlore, brome, méthyle, isopropyle, t-butyle, cyclohexyle, méthoxy, méthylthio, trifluorométhyle, trifluorométhoxy, trifluorométhylthio et

m représente les nombres 0, 1 ou 2.

3. 2-(4-chlorophényl)-3-(4-méthoximinométhyl-phénoxy)-3-méthyl-1-(1,2,4-triazol-1-yl)-2-butanol.

4. 2-(4-chlorophényl)-3-[4-(1-méthoximino-1-éthyl)-phénoxy]-3-méthyl-1-(1,2,4-triazol-1-yl)-2-butanol.

5. Hydroxyalcoyl-azols substitués de formule générale (I)

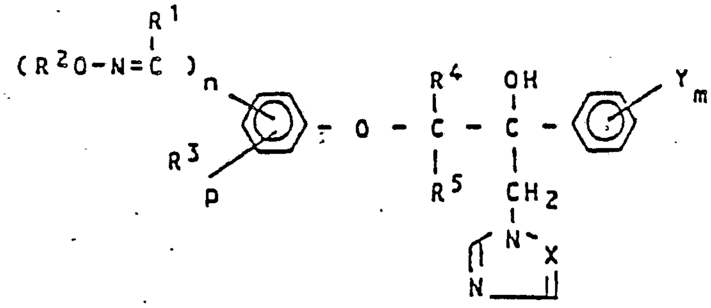

(I)

dans laquelle

$R^1$ représente de l'hydrogène, un alcoyle à chaîne droite ou ramifiée ayant 1 à 10 atomes de carbone, de même qu'un phényle ou benzyle éventuellement substitués chacun une à trois fois, de manière identique ou différente, en envisageant comme substituants les significations de Y ;

$R^2$ représente de l'hydrogène, un alcoyle à chaîne droite ou ramifiée ayant 1 à 10 atomes de carbone, un alcényle ou alcinyle à chaîne droite ou ramifiée ayant chacun 3 à 10 atomes de carbone, de même qu'un phényle et benzyle éventuellement substitués chacun une à trois fois, de manière identique ou différente, en envisageant comme substituants les significations de Y ;

n représente les nombres 1 ou 2 ;

$R^3$ a les significations de Y ;

p signifie les nombres 0, 1 ou 2 ;

$R^4$ représente un alcoyle à chaîne droite ou ramifiée ayant 1 à 4 atomes de carbone ;

$R^5$ un alcoyle à chaîne droite ou ramifiée ayant 1 à 4 atomes de carbone ;

X représente un atome d'azote ou le groupe CH ;

Y de l'halogène, un alcoyle ayant 1 à 4 atomes de carbone, un cycloalcoyle ayant 5 à 7 atomes de carbone, un alcoxy et alcoylthio ayant chacun 1 à 4 atomes de carbone, un halogénoalcoyle et halogénoalcoxy de même que halogénoalcoylthio ayant chacun 1 à 2 atomes de carbone et 1 à 5 atomes d'halogène, identiques ou différents, de préférence des atomes de fluor et de chlore, et

m représente les nombres 0, 1, 2 ou 3, ainsi que leurs sels d'addition d'acides dans le traitement des mycoses.

6. Utilisation d'hydroxyalcoyl-azols substitués de formule générale I

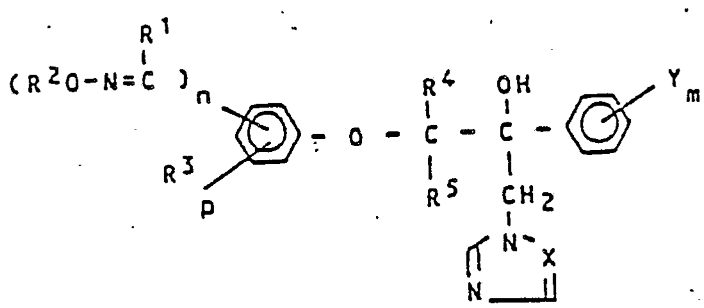

(I)

dans laquelle

$R^1$ représente de l'hydrogène, un alcoyle à chaîne droite ou ramifiée ayant 1 à 10 atomes de carbone de même qu'un phényle ou benzyle éventuellement substitués chacun 1 à 3 fois, de manière identique ou différente, en envisageant comme substituants les significations de Y ;

$R^2$ représente de l'hydrogène, un alcoyle à chaîne droite ou ramifiée ayant 1 à 10 atomes de carbone, un alcényle et alcinyle à chaîne droite ou ramifiée ayant chacun 3 à 10 atomes de carbone, de même qu'un phényle et benzyle éventuellement substitués chacun 1 à 3 fois, de manière identique ou différente, en envisageant comme substituants les significations de Y ;

n représente les nombres 1 ou 2 ;

$R^3$ a les significations de Y ;

p signifie les nombres 0, 1 ou 2 ;

$R^4$ représente un alcoyle à chaîne droite ou ramifiée ayant 1 à 4 atomes de carbone ;

$R^5$ présente un alcoyle à chaîne droite ou ramifiée ayant 1 à 4 atomes de carbone ;

X représente un atome d'azote ou le groupe CH ;

Y représente de l'halogène, un alcoyle ayant 1 à 4 atomes de carbone, un cycloalcoyle ayant 5 à 7 atomes de carbone, un alcoxy et alcoylthio ayant chacun 1 à 4 atomes de carbone, un halogénoalcoyle et halogénoalcoxy de même qu'un halogénoalcoylthio ayant chacun 1 à 2 atomes de carbone et 1 à 5 atomes d'halogène, identiques ou différents, de préférence des atomes de fluor et de chlore ;

m représente les nombres 0, 1, 2 ou 3, ainsi que leurs sels d'addition d'acides dans la préparation de médicaments pour le traitement des mycoses.

7. Procédé de fabrication d'hydroxyalcoylazols substitués de formule générale (I) selon la revendication 1, caractérisé en ce qu'on fait réagir des oxiranes de formule (II)

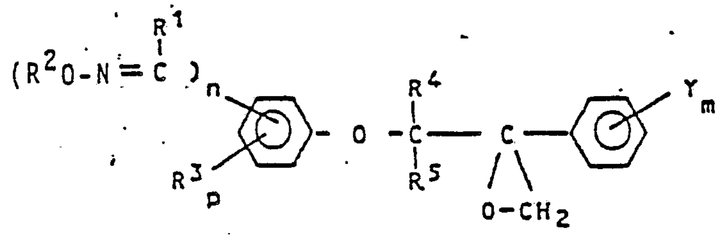

(II)

dans laquelle $R^1$, $R^2$, $R^3$, $R^4$, $R^5$, Y et les indices n, m et p ont la signification indiquée à la revendication 1, avec des azols de formule (III)

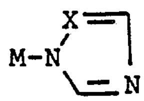

(III)

dans laquelle

X a la signification indiquée à la revendication 1 et

M représente de l'hydrogène ou un métal alcalin, en présence d'un diluant et éventuellement en présence d'une base, et en ce que sur les composés ainsi obtenus, on fixe éventuellement un acide.

8. Médicaments contenant des hydroxyalcoylazols substitués de formule générale (I) selon la revendication 1.

9. Procédé de fabrication d'un médicament, caractérisé en ce qu'on mélange les hydroxyalcoylazols de formule générale (I) selon la revendication 1 avec des substances de support inertes, non toxiques, pharmaceutiquement appropriées.